# EUROPEAN PATENT APPLICATION

(11) **EP 4 560 319 A1**
(43) Date of publication of application: **28.05.2025**
(21) Application number: 23307053.1
(22) Date of filing: 24.11.2023
(51) Int. Cl.: G01N 33/574

(54) **IMMUNE AND TUMOUR CELLS EXPRESSION OF VISTA IN A PANEL OF CANCER INDICATIONS**

(71) Applicant: PIERRE FABRE MEDICAMENT, 81500 Lavaur (FR)
(72) Inventor: LAUNAY, Pierre, 81106 Castres (FR); BONNET, Roland, 81106 Castres (FR)
(74) Representative: Regimbeau

(57) **Abstract**

The present invention provides multiplex assays for improved scoring of tumour tissues stained with an immune checkpoint protein such as VISTA. Methods of ranking multiple tumour tissues and of diagnosing cancer patients are also provided. The methods disclosed are also useful for identifying patient responsive to anticancer therapy.

## Description

### INTRODUCTION

Immunotherapy has been a game-changer in the field of cancer therapy. In order to ensure that an immune inflammatory response is not constantly activated once tumour antigens have stimulated a response, multiple controls or "checkpoints" are in place or activated. VISTA (V-Domain Ig Suppressor of T Cell Activation) is an immune checkpoint protein that maintains T cell and myeloid quiescence and is a promising target for combination cancer immunotherapy (Yuan et al. Trends Immunol. 42(3): 209-227 (2021)).

VISTA has multiple key roles in controlling immune responses, several of which are unique relative to other immune checkpoint molecules. VISTA antagonism 1) induces a shift in MDSC function, reduces MDSC suppressive activity and enhances the potential for improved antitumor effect through macrophage activation, 2) lowers the threshold of activation for quiescent T cells and allows a greater percentage of T cells to respond to neo-antigens, 3) activates NK cells that may result in independent anticancer effects via antibody-directed cellular cytotoxicity, 4) enhances APC maturation to effectively present antigens to induce a T-cell response, and 5) expands and transforms activated and exhausted T cells into effector cells (Martinet al. Front Immunol. 14:1086102 2023)). In patients, high VISTA expression is associated with poor prognosis in multiple tumour indications, and act as a mediator of resistance to immune checkpoint therapies. Thus, VISTA appears as a novel, important immunotherapy target in oncology.

Several antagonistic anti-VISTA antibodies have indeed been described which can be used for the treatment of cancer (ElTanbouly et al. Clin Exp Immunol. 200(2):120-130 (2020); Mehta et al. Sci Rep. 10(1):1 5171 (2020); Yuan et al. Trends Immunol. 42(3): 209-227 (2021); Tagliamento et al. Immunotargets Ther. 10: 185-200 (2021); Thakkar et al. J Immunother Cancer. 10(2): e003382 (2022); WO 2015/097536; WO 2016/094837; WO 2017/181139; WO 2019/183040). These antibodies appear to relieve VISTA-mediated suppression of the anti-cancer response. In particular, an antibody capable of inhibiting VISTA suppression of the anti-tumour immune response, thereby conferring protective anti-tumour immunity, has been identified (WO 2016/094837; WO 2022/229469).

The interplay between VISTA and the components of the TME may be central in predicting the efficacy of anti-VISTA agents currently under development, as well as in the optimal use of these antagonists in combination with other immune-modulating therapies (Martin et al. Front Immunol. 14:1086102 (2023)). VISTA is found in multiple cell types, including myeloid cells, lymphoid cells, and in tumour cells themselves. VISTA is predominantly expressed in the hematopoietic compartment, and particularly within the myeloid lineage. VISTA has also been described on tumour cells at least in human lung, kidney, colorectal, endometrial, and ovarian cancers.

The variability of VISTA expression needs to be better documented to identify indications of interest that could increase the therapeutic response rate. In this regard, the expression of immunoregulatory proteins such as VISTA on individual immune cells within the tumour microenvironment must be robustly characterised. Since immunotherapy has revolutionised the antineoplastic therapy, there is a need for new immunohistochemistry strategies to detect multiple markers simultaneously in order to better understand tumour environment and predict or assess response to immunotherapy.

### SUMMARY OF THE INVENTION

The present disclosure relates to a method of multispectral immunofluorescence imaging of a biological sample. The described method is a multiplexed method which allows direct detection of an immune checkpoint protein simultaneously with target antigens specific for a plurality of cell types.

In a first aspect, the disclosure, provides a multiplex method of scoring a tumour tissue sample stained with a first immune checkpoint protein, comprising:
a) contacting the tissue sample with a primary antibody directed to the first immune checkpoint protein; and
b) contacting the same tissue sample with plurality of antibodies, the plurality of antibodies comprising:
   - at least one antibody directed to a tumour cell-specific marker;
   - at least one antibody directed to a cytotoxic T cell marker;
   - at least one antibody directed to a M2 macrophage marker;
   - at least one antibody directed to a myeloid-derived cell marker;
   - at least one antibody directed to a second immune checkpoint protein;
c) visualising each of the antibodies in the tissue sample with a reagent that generates a detectable signal corresponding to each of the antibodies, wherein each of the detectable signals is distinguishable from the others;
d) quantifying the number of cells expressing VISTA for each of tumour cells, cytotoxic T cells, M2 macrophage, MDSCs, and cells expressing the second immune checkpoint; and
e) scoring the tumour based on this quantification.

Preferably, the immune checkpoint protein is VISTA, CTLA-4, PDL1, PDL2, PD1, B7-H3, B7-H4, BTLA, HVEM, TIGIT, TIM3, GAL9, LAG3, KIR, 2B4, CD160 (also referred to as BY55), CGEN-15049, CHK1 and CHK2 kinases, IDO1, A2aR, or any one of various B7 family ligands. More preferably, the immune checkpoint is VISTA. Preferably, the tumour cell marker is cytokeratin, the cytotoxic T cell marker is CD8, the M2 macrophage marker is CD123, the MDSC marker is CD33, and/or the second immune checkpoint protein is PD-L1.

In an embodiment, the tumour sample is a formalin-fixed paraffin embedded (FFPE) sample. preferably, the tumour is a solid tumour.

In an embodiment, the antibody is labelled with a detectable label, preferably a fluorescent label.

Advantageously, step c) involves measuring information corresponding to one or more stains of each of the labelled antibodies directed against VISTA, tumour cell-specific marker, the cytotoxic T cell marker, the M2 macrophage marker, the myeloid-derived cell marker, and the immune checkpoint marker in the biological sample. Preferably, step c) involves:
- generating a first image of the biological sample, wherein the first image corresponds to a pattern of VISTA staining in the biological sample;
- generating a second image of the biological sample, wherein the second image corresponds to a pattern of the tumour cell-specific marker in the biological sample;
- generating a third image of the biological sample, wherein the third image corresponds to a pattern of the cytotoxic T cell marker staining in the biological sample;
- generating a fourth image of the biological sample, wherein the fourth image corresponds to a pattern of the M2 macrophage marker staining in the biological sample;
- generating a fifth image of the biological sample, wherein the fifth image corresponds to a pattern of myeloid-derived cell marker staining in the biological sample; and
- generating a sixth image of the biological sample, wherein the sixth image corresponds to a pattern of immune checkpoint marker staining in the biological sample.

In another aspect, the disclosure relates to a method for ranking a plurality of tissue samples, comprising the steps of:
a) scoring each of the tissue samples using the scoring method as described above, and
b) ranking the tissue samples based on the scoring of a);
wherein the samples are from the same tissues from different patients, from different tissues from different patients, or from different tissues from the same patient.

In another aspect, the disclosure relates to a method of prognosing an anticancer therapy in patient comprising:
a) taking a first tumour tissue sample from the patient at a first time point;
b) taking a second tumour tissue sample from the patient at a second time point;
c) scoring the first tumour sample from patient using the scoring method as described, thereby generating a first score;
d) scoring the second tumour sample from the patient using the scoring method as described, thereby generating a second score;
e) comparing the first score of c) and the second score of d), and
f) determining the prognosis of the patient based on the comparison of e).
in an embodiment, the prognosis of the patient is bad when the second score of d) is higher than the first score of c). In an embodiment, the anticancer therapy is immunotherapy or chemotherapy.

In another aspect, the disclosure relates to a method for predicting a treatment response of a patient to a proposed treatment for a cancer comprising:
a) scoring a tumour tissue sample from the patient using the scoring method as described, and
b) determining that the patient will or will not be responsive to the proposed treatment based on the score of the tumour tissue sample.

In an instance, the patient is predicted to be responsive to the treatment when at least one of tumour cells, cytotoxic T cells, M2 macrophages, and MDSCs expresses VISTA in the tumour tissue sample.

Preferably, the treatment comprises administering an anti-VISTA monoclonal antibody 26A comprising 6 CDRs represented by SEQ ID NOS:3-8. More preferably, the anti-VISTA monoclonal antibody 26A comprises the V_{H} and V_{L} represented by SEQ ID NO:9 and SEQ ID NO:10.

### LEGENDS OF THE FIGURES

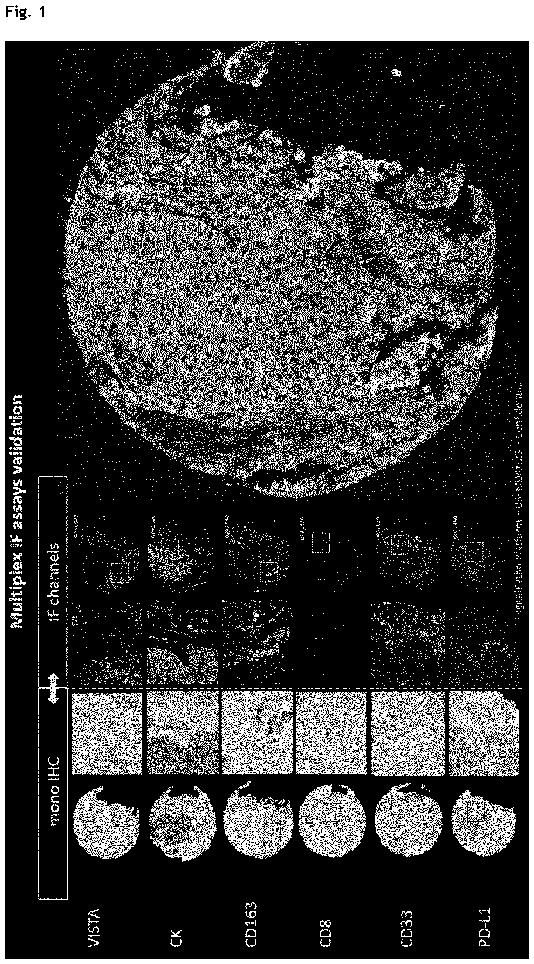

**Figure1****:** Validation of multiplex panel VISTA, CK, CD163, CD8, CD33 on lung squamous cell carcinoma tissue. Individual mono IHC staining was performed on serial section and compared to their respective IF channels.

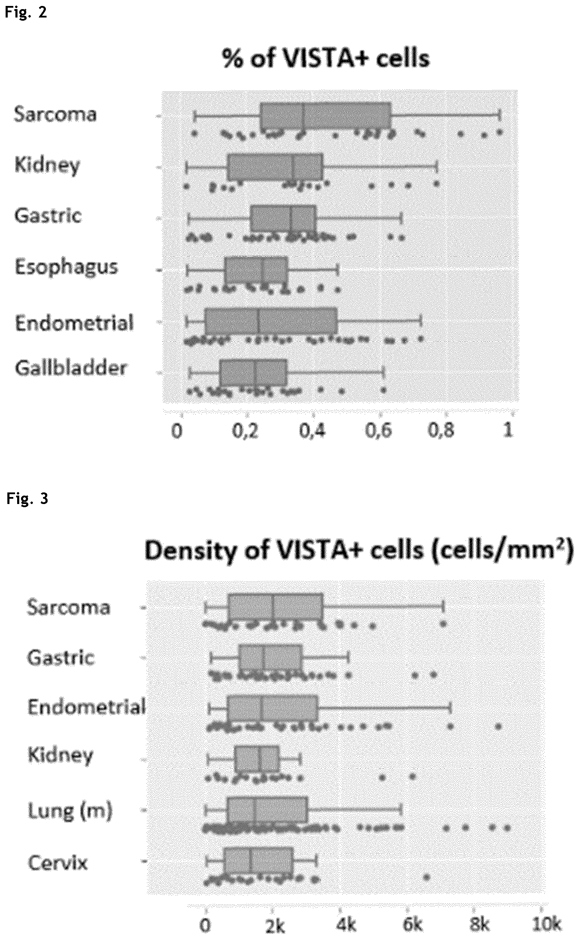

**Figure 2****:** Mean % of VISTA positive cells in solid tumour indications.
**Figure 3****:** Mean Density of VISTA positive cells in solid tumour indications.

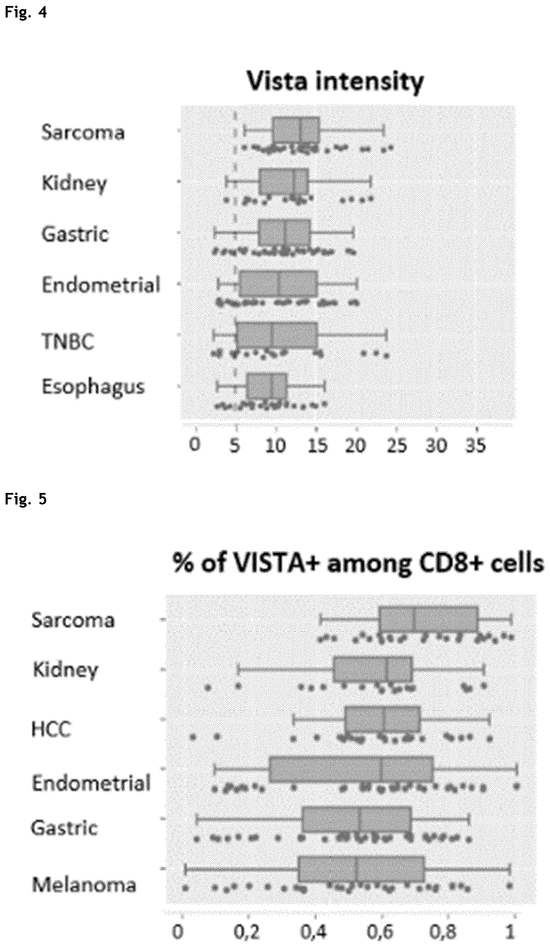

**Figure 4****:** Mean Intensity value of VISTA fluorescence signal in solid tumour indications.
**Figure 5****:** Mean % of VISTA positive cells in CD8 cell population.

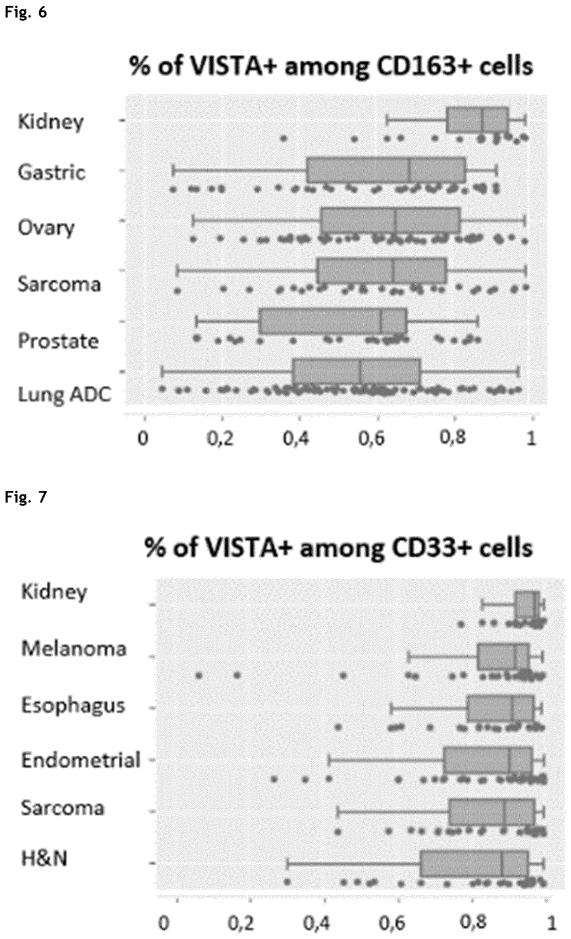

**Figure 6****:** Mean % of VISTA positive cells in CD163 cell population.
**Figure 7****:** Mean % of VISTA positive cells in CD33 cell population.

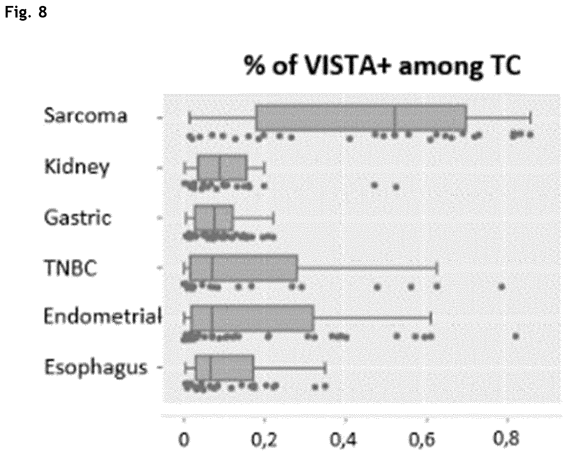

**Figure 8****:** Mean % of VISTA positive cells in Tumour Cell population.

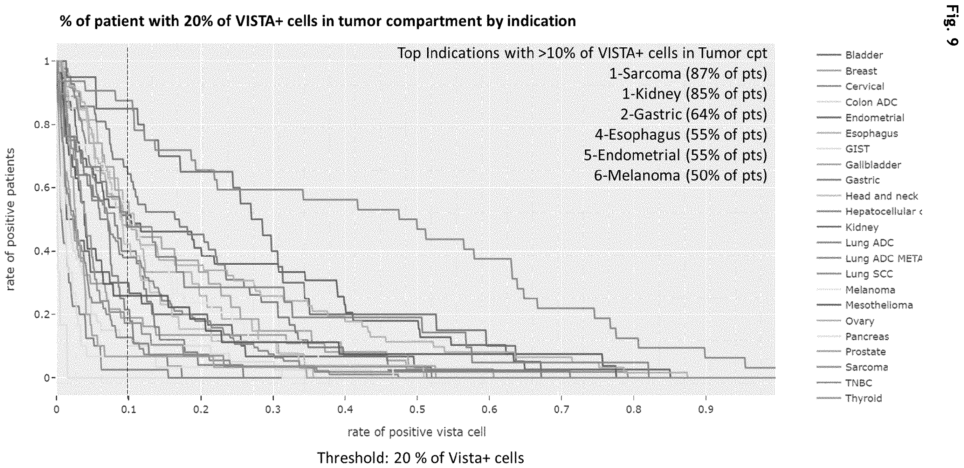

**Figure 9****:** % of patient depending of the % VISTA positive cells in the tumour compartment of different tumour solid indications

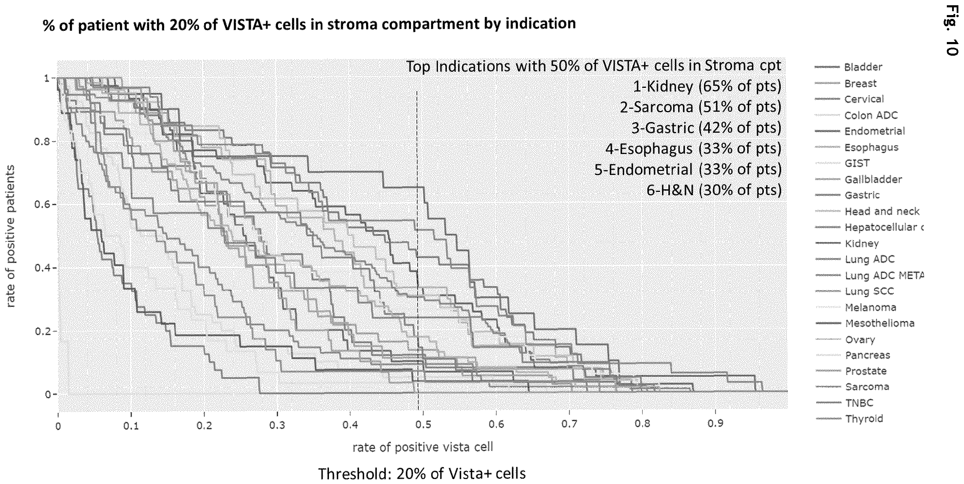

**Figure 10****:** % of patient depending of the % VISTA positive cells in the stroma compartment of different tumour solid indications

### DESCRIPTION OF THE INVENTION

All methods and materials similar or equivalent to those described herein can be used in the practice or testing of the present invention, with suitable methods and materials being described herein. The practice of the invention employs, unless other otherwise indicated, conventional techniques or protein chemistry, molecular virology, microbiology, recombinant DNA technology, and pharmacology, which are within the skill of the art. Such techniques are explained fully in the literature (see *e.g*., Ausubel et al., Short Protocols in Molecular Biology, Current Protocols; 5th Ed., 2002; Remington's Pharmaceutical Sciences, 17th ed., Mack Publishing Co., Easton, Pa., 1985; and Sambrook et al., Molecular Cloning: A Laboratory Manual, Cold Spring Harbor Laboratory Press; 3rd Ed., 2001). The nomenclatures used in connection with, and the laboratory procedures and techniques of, molecular and cellular biology, protein biochemistry, enzymology and medicinal and pharmaceutical chemistry described herein are those well-known and commonly used in the art. All publications, patent applications, patents, and other references mentioned herein are incorporated by reference in their entirety. Further, the materials, methods, and examples are illustrative only and are not intended to be limiting, unless otherwise specified.

The present invention will become more fully understood from the detailed description given herein and from the accompanying drawings, which are given by way of illustration only and do not limit the intended scope of the invention.

### Definitions

Unless specifically defined, all technical and scientific terms used herein have the same meaning as commonly understood by a skilled artisan in chemistry, biochemistry, cellular biology, molecular biology, and medical sciences.

The term **"about"** or **"approximately"** refers to the normal range of error for a given value or range known to the person of skills in the art. It usually means within 20%, such as within 10%, or within 5% (or 1% or less) of a given value or range.

The terms **"antibody"** and **"immunoglobulin"** or **"Ig"** are used interchangeably herein. These terms are used herein in the broadest sense and specifically cover monoclonal antibodies (including full length monoclonal antibodies) of any isotype such as IgG, IgM, IgA, IgD, and IgE, polyclonal antibodies, multispecific antibodies, chimeric antibodies, and antibody fragments, provided that said fragments retain the desired biological function. These terms are intended to include a polypeptide product of B cells within the immunoglobulin class of polypeptides that is capable of binding to a specific molecular antigen and is composed of two identical pairs of polypeptide chains inter-connected by disulfide bonds, wherein each pair has one heavy chain (about 50-70 kDa) and one light chain (about 25 kDa) and each amino-terminal portion of each chain includes a variable region of about 100 to about 130 or more amino acids and each carboxy-terminal portion of each chain includes a constant region (See, Borrebaeck (ed.) (1995) Antibody Engineering, Second Ed., Oxford University Press.; Kuby (1997) Immunology, Third Ed., W.H. Freeman and Company, New York). Each variable region of each heavy and light chain is composed of three complementarity-determining regions (CDRs), which are also known as hypervariable regions and four frameworks (FRs), the more highly conserved portions of variable domains, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. In some embodiments, the specific molecular antigen can be bound by an antibody provided herein includes the target VISTA polypeptide, fragment or epitope. An antibody reactive with a specific antigen can be generated by recombinant methods such as selection of libraries of recombinant antibodies in phage or similar vectors, or by immunising an animal with the antigen or an antigen-encoding nucleic acid.

Antibodies also include, but are not limited to, synthetic antibodies, monoclonal antibodies, recombinantly produced antibodies, multispecific antibodies (including bispecific antibodies), human antibodies, humanised antibodies, camelised antibodies, chimeric antibodies, intrabodies, anti-idiotypic (anti-Id) antibodies, and functional fragments of any of the above, which refers a portion of an antibody heavy or light chain polypeptide that retains some or all of the biological function of the antibody from which the fragment was derived. The antibodies provided herein can be of any type (*e.g*., IgG, IgE, IgM, IgD, IgA and IgY), any class (*e.g.*, IgG1, IgG2, IgG3, IgG4, IgA1 and IgA2), or any subclass (*e.g*., IgG2a and IgG2b) of immunoglobulin molecule.

An **"antigen"** is a predetermined antigen to which an antibody can selectively bind. The target antigen may be a polypeptide, carbohydrate, nucleic acid, lipid, hapten or other naturally occurring or synthetic compound. In some embodiments, the target antigen is a polypeptide, including, for example, a VISTA polypeptide.

The terms **"binds"** or **"binding"** as used herein refer to an interaction between molecules to form a complex which, under physiologic conditions, is relatively stable. Interactions can be, for example, non-covalent interactions including hydrogen bonds, ionic bonds, hydrophobic interactions, and/or van der Waals interactions. A complex can also include the binding of two or more molecules held together by covalent or non-covalent bonds, interactions or forces. The strength of the total non-covalent interactions between a single antigen-binding site on an antibody and a single epitope of a target molecule, such as VISTA, is the affinity of the antibody or functional fragment for that epitope. The ratio of association (*k₁*) to dissociation (*k*_{-*1*}) of an antibody to a monovalent antigen (*k₁*/ *k*_{-*1*}) is the association constant *K*, which is a measure of affinity. The value of K varies for different complexes of antibody and antigen and depends on both k*₁* and *k*_{-*1*}. The association constant *K* for an antibody provided herein can be determined using any method provided herein or any other method well known to those skilled in the art. The affinity at one binding site does not always reflect the true strength of the interaction between an antibody and an antigen. When complex antigens containing multiple, repeating antigenic determinants, such as a polyvalent VISTA, come in contact with antibodies containing multiple binding sites, the interaction of antibody with antigen at one site will increase the probability of a reaction at a second site. The strength of such multiple interactions between a multivalent antibody and antigen is called the avidity. The avidity of an antibody can be a better measure of its binding capacity than is the affinity of its individual binding sites. For example, high avidity can compensate for low affinity as is sometimes found for pentameric IgM antibodies, which can have a lower affinity than IgG, but the high avidity of IgM, resulting from its multivalence, enables it to bind antigen effectively. Methods for determining whether two molecules bind are well known in the art and include, for example, equilibrium dialysis, surface plasmon resonance, and the like. In a particular embodiment, said antibody, or antigen-binding fragment thereof, binds to VISTA with an affinity that is at least twofold greater than its affinity for binding to a non-specific molecule such as BSA or casein. In a more particular embodiment, said antibody, or antigen-binding fragment thereof, binds only to VISTA.

As used herein, the term **"biological sample"** or **"sample"** refers to a sample that has been obtained from a biological source, such as a patient or subject. A "biological sample" as used herein refers notably to a whole organism or a subset of its tissues, cells or component parts (e.g., blood vessel, including artery, vein and capillary, body fluids, including but not limited to blood, serum, mucus, lymphatic fluid, synovial fluid, cerebrospinal fluid, saliva, amniotic fluid, amniotic cord blood, urine, vaginal fluid and semen). **"Biological sample"** further refers to a homogenate, lysate or extract prepared from a whole organism or a subset of its tissues, cells or component parts, or a fraction or portion thereof. Lastly, "biological sample" refers to a medium, such as a nutrient broth or gel in which an organism has been propagated, which contains cellular components, such as proteins or nucleic acid molecules.

As used herein, the term **"biomarker"** or **"marker"** is intended to encompass a biochemical characteristic that is used as an indicator of a biologic state and includes genes (and nucleotide sequences of such genes), mRNAs (and nucleotide sequences of such mRNAs) and proteins (and amino acid sequences of such proteins) and post-translationally modified forms of proteins (i.e., phosphorylated and non-phosphorylated forms). A biomarker may notably refer to a substance that can be used to diagnose, or to measure the progress of a disease or condition, or the effects of treatment of a disease or condition is meant. A biomarker can be, for example, the presence of a nucleic acid, protein, or antibody associated with the presence of cancer or another disease in an individual. A **"biomarker expression pattern"** is intended to refer to a quantitative or qualitative summary of the expression of one or more biomarkers in a subject, such as in comparison to a standard or a control.

The terms **"cell proliferative disorder"** and **"proliferative disorder"** refer to disorders that are associated with some degree of abnormal cell proliferation. In some embodiments, the cell proliferative disorder is a tumour or cancer. **"Tumour",** as used herein, refers to all neoplastic cell growth and proliferation, whether malignant or benign, and all pre-cancerous and cancerous cells and tissues. The terms **"cancer", "cancerous", "cell proliferative disorder", "proliferative disorder",** and **"tumour"** are not mutually exclusive as referred to herein. The terms **"cancer"** and **"cancerous"** refer to or describe the physiological condition in mammals that is typically characterised by unregulated cell growth. A **"cancer"** as used herein is any malignant neoplasm resulting from the undesired growth, the invasion, and under certain conditions metastasis of impaired cells in an organism. The cells giving rise to cancer are genetically impaired and have usually lost their ability to control cell division, cell migration behaviour, differentiation status and/or cell death machinery. Most cancers form a tumour but some haematopoietic cancers, such as leukaemia, do not. Thus, a **"cancer"** as used herein may include both benign and malignant cancers.

As used herein, a **"CDR"** refers to one of three hypervariable regions (H1, H2 or H3) within the non-framework region of the immunoglobulin (Ig or antibody) VH β-sheet framework, or one of three hypervariable regions (L1, L2 or L3) within the non-framework region of the antibody VL β-sheet framework. Accordingly, CDRs are variable region sequences interspersed within the framework region sequences.

A **"chemotherapeutic agent"** is a chemical or biological agent (*e.g*., an agent, including a small molecule drug or biologic, such as an antibody or cell) useful in the treatment of cancer, regardless of mechanism of action. Chemotherapeutic agents include compounds used in targeted therapy and conventional chemotherapy. Chemotherapeutic agents include, but are not limited to, alkylating agents, antimetabolites, anti-tumour antibiotics, mitotic inhibitors, chromatin function inhibitors, anti-angiogenesis agents, anti-oestrogens, anti-androgens or immunomodulators.

As used herein, the term **"colour"** refers to a colour represented in a fluorescence image that directly correlates with the fluorescence emission spectra of the detectable label bound to an antibody when the antibody is specifically bound to a target antigen in situ in a tumour tissue sample.

The term **"decreased",** as used herein, refers to the level of a biomarker, e.g., VISTA, of a subject at least 1-fold (e.g., 1, 2, 3, 4, 5, 10, 20, 30, 40, 50, 60, 70, 80, 90, 100, 1000, 10,000- fold or more) lower than its reference value. "Decreased", as it refers to the level of a biomarker, e.g., VISTA, of a subject, signifies also at least 5% lower (e.g., 5%, 6%, 7%, 8%, 9%, 10%, 15%, 20%, 25%, 30%, 35%, 40%, 45%, 50%, 55%, 60%, 65%, 70%, 75%, 80%, 85%, 90%), 95%), 99%), or 100%) than the level in the reference sample or with respect to the reference value for said marker.

The term **"detecting"** as used herein encompasses quantitative or qualitative detection.

The term **"detectable probe"** or **"detectable agent",** as used herein, refers to a composition that provides a detectable signal. The term refers to a substance that can be used to ascertain the existence or presence of a desired molecule, such as an antibody provided herein, in a sample or subject. A detectable agent can be a substance that is capable of being visualised or a substance that is otherwise able to be determined and/or measured (*e.g*., by quantitation). The term includes, without limitation, any fluorophore, chromophore, radiolabel, enzyme, antibody or antibody fragment, and the like, that provide a detectable signal via its activity.

As used herein, **"diagnosis"** or **"identifying a subject having"** refers to a process of identifying a disease, condition, or injury from its signs and symptoms. A diagnosis is notably a process of determining if an individual is afflicted with a disease or ailment (e.g., cancer). Cancer is diagnosed for example by detecting either the presence of a marker associated with cancer such as, e.g., VISTA.

The term **"diagnostic agent"** refers to a substance administered to a subject that aids in the diagnosis of a disease. Such substances can be used to reveal, pinpoint, and/or define the localisation of a disease-causing process. In some embodiments, a diagnostic agent includes a substance that is conjugated to an antibody provided herein, that when administered to a subject or contacted to a sample from a subject, aids in the diagnosis of cancer, tumour formation, or any other cell proliferative disease, disorder or condition.

As used herein, the term **"formalin-fixed paraffin embedded (FFPE) tissue section"** refers to a piece of tissue, e.g., a biopsy that has been obtained from a subject, fixed in formaldehyde (e.g., 3%-5% formaldehyde in phosphate buffered saline) or Bouin solution, embedded in wax, cut into thin sections, and then mounted on a microscope slide.

The term **"immune cell",** as used herein, refers broadly to cells that are of haematopoietic origin and have a role in the immune response. Immune cells include lymphocytes, such as B cells and T cells; natural killer cells; and myeloid cells, such as monocytes, macrophages, eosinophils, mast cells, basophils, and granulocytes.

As used herein, the terms **"immune checkpoint"** or **"immune checkpoint protein"** refer to certain proteins made by some types of immune system cells, such as T cells, and some cancer cells. Such proteins regulate T cell function in the immune system. Notably, they help keep immune responses in check and can keep T cells from killing cancer cells. Said immune checkpoint proteins achieve this result by interacting with specific ligands which send a signal into the T cell and essentially switch off or inhibit T cell function. Inhibition of these proteins results in restoration of T cell function and an immune response to the cancer cells. Examples of checkpoint proteins include, but are not limited to CTLA-4, PDL1, PDL2, PD1, B7-H3, B7-H4, BTLA, HVEM, TIGIT, TIM3, GAL9, LAG3, VISTA, KIR, 2B4 (belongs to the CD2 family of molecules and is expressed on all NK, γδ, and memory CD8+ (αβ) T cells), CD160 (also referred to as BY55), CGEN-15049, CHK1 and CHK2 kinases, IDO1, A2aR, and various B7 family ligands.

The term **"increased",** as used herein, refers to the level of a biomarker, e.g., VSIG4, of a subject at least 1-fold (e.g., 1, 2, 3, 4, 5, 10, 20, 30, 40, 50, 60, 70, 80, 90, 100, 1000, 10,000-fold or more) greater than its reference value. "Increased", as it refers to the level of a biomarker, e.g., VISTA, of a subject, signifies also at least 5% greater (e.g., 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, or 100%) than the level in the reference sample or with respect to the reference value for said marker.

A **"macrophage"** as used herein is a myeloid immune cell which is part of the mononuclear phagocyte system. Macrophages are present in virtually all tissues of the body, where they differentiate into tissue-resident macrophages, thus forming a network of specialised cells, including alveolar macrophages (lung), red pulp macrophages (spleen), Kupffer cells (liver), microglia (brain), Langerhans cells (epidermis), osteoclasts (bone), and histiocytes (connective tissue). Macrophages are phagocytes and antigen presenting cells that differentiate from monocytes in circulating peripheral blood. They also produce many important cytokines at all stages of the immune response, including the tissue repair phase. They play an important role in both innate and adaptive immunity by activating T lymphocytes.

Mature macrophages can be divided into two populations, according to their activation state and functions, i.e., M1-type (classically activated macrophage) and M2-type (alternatively activated macrophage). M1 macrophages, also referred to as "killer macrophages", are characterised by the production of high levels of proinflammatory cytokines, an ability to mediate resistance to pathogens, strong microbicidal properties, high production of reactive nitrogen and oxygen intermediates, and promotion of Th1 responses. They can express one or more of CD80, CD86, CD197, HLA-DR, and CD40 on their cell membrane; advantageously they express more than one of these markers. M1 polarisation is triggered by LPS, IFN-γ and granulocyte-macrophage colony stimulating factor (GM-CSF), and results in secretion of proinflammatory cytokines such as IL-1β, TNF-α, IL-12p70, IL-6, IL-18 and IL-23.

Macrophages that activate Th2 T lymphocytes provide an anti-inflammatory response and are denoted M2 macrophages. M2 macrophage activation is induced by fungal cells, immune complexes, helminth infections, complement components, apoptotic cells, macrophage colony stimulating factor (MCSF), IL-4, IL-13, IL-10 and TGF-B. M2 macrophages have an important role in wound healing and tissue repair. In addition, they have pro-tumoural functions, including e.g., tumour invasion, metastasis, tumour cell proliferation, tumour growth, tumour survival, neo-angiogenesis, suppression of adaptive or innate immunity and extracellular matrix remodelling. M2 macrophages mainly secrete Arginase-I, IL-10 and TGF-B and other anti-inflammatory cytokines, which have the function of reducing inflammation and contributing to tumour growth and immunosuppressive function. Cell surface markers associated with the M2 phenotype include CD163, CD206, CD200R, CD209. M2 macrophages thus express one or more of these markers; advantageously they express more than one of these markers.

As used herein, the term **"multispectral imaging"** and grammatical variations thereof refers to the labelling and imaging of a tumour tissue sample on a slide or any other suitable support using a multispectral scanner. A **"multispectral scanner"** as used herein refers to a device that is able to collect data over a variety of different wavelength ranges. As used herein, a **"multispectral image"** refers to a collection of a few image layers of the same tumour tissue sample, each of them acquired at a particular wavelength band

As used herein, the term **"multiplex"** refers to the use of more than one label for the simultaneous or sequential detection and measurement of biologically active material.

As used herein, the term **"myeloid derived suppressor cell"** or **"MDSC"** generally refers to a group of heterogeneous cells of immature hematopoietic myeloid-cell progenitors which commonly express CD33, CD11b and CD45. Various subpopulations of MDSCs have been defined. For example, monocytic-MDSCs (M-MDSCs) are commonly associated with expression of CD14 and CD124 and low expression of HLA-DR. In some embodiments, the MDSC population is an MO-MDSC population. Polymorphonuclear MDSCs (PMN-MDSCs) are associated with expression of CD15, CD66b, and CD124, and no expression of HLA-DR. Immature MDSCs (I- MDSCs) are associated with expression of CD117 and CD34 and no expression of LIN and HLA-DR. See e.g., Ugel et al. (2015) JCI Vol 125 (9), page 3365.

MDSCs, as used herein, are immune cells which exploit a plethora of redundant mechanisms to exert immunosuppressive functions and therefore negatively regulate immune responses. For example, MDSC are potent inhibitors of T and B lymphocytes activation, proliferation and responses, in particular by nutrients (e.g. L-arginine and L-cysteine) depletion mechanisms, the production of reactive oxygen species (ROS) and reactive nitrogen species (RNS), perturbation of T-lymphocyte trafficking (e.g. L-selectin expression decrease and aberrant chemokine release), induction of apoptosis (via Galectin 9) and by deviating T-lymphocytes differentiation towards Th-17 responses through IL-1 6 production. MDSC have also the capacity to expand antigen-specific natural regulatory T cells (nTreg), to promote conversion of naive T cells into induced Treg (iTreg) cells and to promote Treg infiltration at inflamed, infected or tumour sites. MDSC were also described to decrease the number and inhibit function of NK cells, in particular by membrane bound TGFB. Furthermore, MDSC skew macrophages towards an M2 phenotype (non-inflammatory macrophages) by inhibiting macrophages production of IL-12. Similarly, MDSC impair dendritic cell (DC) function by producing IL-10, which also inhibits IL-12 production by DC and reduces DC capacity to activate T cells. Finally, MDSC act on non-haematopoietic cell and have been in particular widely recognised to facilitate tumour angiogenesis, tumour spread, tumour-cell invasion and metastasis.

As used herein, a **"stain"** is a compound that binds to a biological sample and generates a measurable signal in the sample which can be used to determine where the stain is located in the sample. A stain can be a fluorescent compound or moiety that generates fluorescence emission when exposed to radiation. Fluorescent compounds or moieties are also interchangeably referred to as fluorescent "labels". A stain can be a chromogenic compound or moiety that absorbs certain wavelengths of incident radiation and does not absorb other wavelengths of incident radiation. A stain can be non-specific, such that it does not bind or localise with a particular type of target molecule, structure, or compartment within a sample. Examples of such non-specific stains are "counterstains". A stain can be specific, and can bind to or localise with a particular type of target molecule, structure, or compartment within a sample. Examples of specific stains are DAPI (which binds to DNA), antibody-linked stains (e.g., fluorescent moieties linked directly or indirectly to antibodies that bind specifically to particular antigens such as tumour markers in a sample), and nucleic acid-linked stains (e.g., fluorescent moieties linked directly or indirectly to oligonucleotides that bind specifically to particular nucleic acid targets in a sample, such as different RNA species).

As used herein, the term **"T cells"** refer to a type of lymphocytes (white blood cells) that have a central role in cell-mediated immunity. T cells can be distinguished from other lymphocytes by the presence of a T cell receptor (TCR) on the cell surface. T cell types include T helper cells (Th cells), memory T cells (Tcm, Tern or Temra), regulatory T cells (Treg), cytotoxic T cells (CTL), natural killer T cells (NKT cells), gamma delta T cells and mucosa-associated invariant T cells (MAIT). In particular, "CD8+ T cells", also known as "cytotoxic T cells", have the main function of directly killing intracellular pathogens and eliminating mutated and cancerous cells. These T cells are characterised by the expression CD8. "CD4+ T cells" or "T helper cells" positively or negatively regulate other immune cells. These cells express the CD4 glycoprotein on their surface. "Regulatory T cells" or "Tregs" are a population of T cells that express the FOXP3 transcription factor and maintain immunological tolerance. Tregs cells are important for maintaining homeostasis, controlling the magnitude and duration of the inflammatory response and preventing autoimmune and allergic responses. During an immune response, Tregs thus suppress immune reactions mediated by effector T cells, such as CD4+ or CD8+ effector T cells.

As used herein, the term **"therapeutic agent"** refers to any agent that can be used in treating, preventing or alleviating a disease, disorder or condition, including in the treatment, prevention or alleviation of one or more symptoms of a VISTA-mediated disease, disorder, or condition and/or a symptom related thereto. In some embodiments, a therapeutic agent refers to an anti-VISTA antibody provided herein. In some embodiments, a therapeutic agent refers to an agent other than an anti-VISTA antibody provided herein. In some embodiments, a therapeutic agent is an agent which is known to be useful for, or has been or is currently being used for the treatment, prevention or alleviation of one or more symptoms of a VISTA-mediated disease, disorder, condition, and/or a symptom related thereto.

The combination of therapies (*e.g*., use of therapeutic agents) can be more effective than the additive effects of any two or more single therapies. For example, a synergistic effect of a combination of therapeutic agents permits the use of lower dosages of one or more of the agents and/or less frequent administration of the agents to a subject with a VISTA-mediated disease, disorder or condition and/or a symptom related thereto. The ability to utilise lower dosages of therapeutic therapies and/or to administer the therapies less frequently reduces the toxicity associated with the administration of the therapies to a subject without reducing the efficacy of the therapies in the prevention, treatment or alleviation of one or more symptoms of a VISTA-mediated disease, disorder or condition and/or a symptom related thereto. In addition, a synergistic effect can result in improved efficacy of therapies in the prevention, treatment or alleviation of one or more symptoms of a VISTA-mediated disease, disorder or condition and/or a symptom related thereto. Finally, synergistic effect of a combination of therapies (*e.g.*, therapeutic agents) may avoid or reduce adverse or unwanted side effects associated with the use of any single therapy.

The term **"tumour microenvironment"** or **"TME"** refers to the cellular environment in which a tumour exists. A tumour microenvironment can include surrounding blood vessels, immune cells, fibroblasts, bone marrow-derived inflammatory cells, lymphocytes, signalling molecules and the extracellular matrix.

The term **"VISTA"** or **"VISTA polypeptide"** and similar terms refers to the polypeptide ("polypeptide," "peptide" and "protein" are used interchangeably herein) encoded by the human Chromosome 10 Open Reading Frame 54 (VISTA) gene, which is also known in the art as B7-H5, platelet receptor Gi24, Gl24, Stress Induced Secreted Protein1, SISP1, and PP2135, for example, comprising the amino acid sequence of SEQ ID NO: 1, and related polypeptides, including SNP variants thereof. The VISTA polypeptide has been shown to or is predicted to comprise several distinct regions within the amino acid sequence including: a signal sequence (residues 1-32; *see* Zhang et al., Protein Sci. 13:2819-2824 (2004)); an immunoglobulin domain - IgV-like (residues 33-162); and a transmembrane region (residues 195-215). The mature VISTA protein includes amino acid residues 33-311 of SEQ ID NO: 1. The extracellular domain of the VISTA protein includes amino acid residues 33-194 of SEQ ID NO: 1. Related polypeptides include allelic variants (*e.g.*, SNP variants); splice variants; fragments; derivatives; substitution, deletion, and insertion variants; fusion polypeptides; and interspecies homologs, preferably, which retain VISTA activity and/or are sufficient to generate an anti-VISTA immune response. VISTA can exist in a native or denatured form. The VISTA polypeptides described herein may be isolated from a variety of sources, such as from human tissue types or from another source, or prepared by recombinant or synthetic methods. A "native sequence VISTA polypeptide" comprises a polypeptide having the same amino acid sequence as the corresponding VISTA polypeptide derived from nature. Such native sequence VISTA polypeptides can be isolated from nature or can be produced by recombinant or synthetic means. The term "native sequence VISTA polypeptide" specifically encompasses naturally-occurring truncated or secreted forms of the specific VISTA polypeptide (*e.g*., an extracellular domain sequence), naturally-occurring variant forms (*e.g.*, alternatively spliced forms) and naturally-occurring allelic variants of the polypeptide.

A cDNA nucleic acid sequence encoding the VISTA polypeptide, for example, comprises the nucleic acid sequence put forth in SEQ ID NO: 2.

VISTA is predominantly expressed on the myeloid cell population, particularly myeloid-derived suppressor cells (MDSCs), neutrophils, monocytes, macrophages, and dendritic cells. VISTA can also be expressed on regulatory T cells and CD4⁺ naïve T lymphocytes. As described herein, VISTA is an immunomodulator, that is a negative checkpoint regulator of immune responses (*e.g*., inhibits or suppresses immune responses). VISTA has been identified as a negative checkpoint regulator of T cell function and is known to suppress autoimmune responses in a variety of human and mouse models of autoimmunity. VISTA has in particular been shown to promote tumourigenesis, block T cell function, and modulate the activity of macrophages and immunosuppressive myeloid-derived suppressor cells (MDSCs). VISTA is upregulated on immunosuppressive tumour infiltrating leukocytes such as inhibitory regulatory T cells (Tregs) and MDSCs. The presence of VISTA in the tumour microenvironment hinders effective T cell responses and has been implicated in a number of human cancers including prostate, colon, skin, pancreatic, and lung (ElTanbouly et al. Clin Exp Immunol. 200(2):120-130 (2020); Mehta et al. Sci Rep. 10(1):1 5171 (2020); Yuan et al. Trends Immunol. 42(3): 209-227 (2021); Tagliamento et al. Immunotargets Ther. 10: 185-200 (2021); Thakkar et al. J Immunother Cancer. 10(2): e003382 (2022); WO 2015/097536; WO 2016/094837; WO 2017/181139; WO 2019/183040)

Orthologs to the VISTA polypeptide are also well known in the art. For example, the mouse ortholog to the VISTA polypeptide is V-region Immunoglobulin-containing Suppressor of T cell Activation (VISTA) (also known as PD-L3, PD-1H, PD-XL, Pro1412 and UNQ730), which shares approximately 70% sequence identity to the human polypeptide. Orthologs of VISTA can also be found in additional organisms including chimpanzee, cow, rat and zebrafish.

### Multiplex assays

The present invention features multiplex assays for improved scoring of tumour tissues stained with an immune checkpoint protein. These assays are particularly useful because they enable deeper interrogation of the intricate biology within the tumour microenvironment than the assays of the prior art, including detection of cell-to-cell spatial interactions that correlate with clinical outcomes. It also provides a practical path for generating reliable and reproducible results in a clinically suitable, high-throughput workflow.

The multiplex assays described herein comprise detecting the cells expressing the immune checkpoint protein, in combination with the detection of at least one marker of at least one specific cell type, e.g., a tumour cell or an immune cell, in the tumour tissue, quantifying the expression of the cells expressing the immune checkpoint protein in cells expressing the at least one marker of the at least one specific cell type, and scoring the tumour tissue based on the expression of the immune checkpoint protein in the cell type.

Preferably, the immune checkpoint protein is VISTA, CTLA-4, PDL1, PDL2, PD1, B7-H3, B7-H4, BTLA, HVEM, TIGIT, TIM3, GAL9, LAG3, KIR, 2B4, CD160 (also referred to as BY55), CGEN-15049, CHK1 and CHK2 kinases, IDO1, A2aR, or any one of various B7 family ligands. More preferably, the immune checkpoint is VISTA.

These assays thus allow identifying the various cell types which express the immune checkpoint protein, preferably VISTA, in a tumour sample. In particular, these assays allow the skilled person to identify which of the cell types known to be involved in immunosuppressive pathway by the immune checkpoint protein, e.g., VISTA, actually express the immune checkpoint protein, e.g., VISTA, in the tumour sample. The spatial biology revealed by the present assays, including cellular protein coexpression, localisation, and arrangement, is particularly important for designing or adapting a treatment plan for a cancer patient. Determining or predicting a patient's likelihood to respond to a specific therapy is indeed aided by the information provided by the present assays, i.e., detailed cell-level evaluation of the TME-specific cell presence, their functional status, and how they interact within the TME.

Preferably, the multiplex assay described herein comprises detecting the expression of the immune checkpoint protein, preferably VISTA, in multiple cell types in the tumour tissue. More preferably, the assay involves detecting the expression of the immune checkpoint protein, preferably VISTA, in a tumour cell and in an immune cell, thereby allowing discriminating between the two. Advantageously, the assay involves detecting the expression of the immune checkpoint protein, preferably VISTA, in more than one type of immune cells. For example, the expression of the immune checkpoint protein, notably VISTA, can be detected in cytotoxic T cells, M2 macrophages, myeloid-derived cells (MDSCs), etc. Alternatively, the assay involves detecting the expression of the immune checkpoint protein, preferably VISTA, in a tumour cell and a cell expressing a second immune checkpoint protein, such as a cell expressing PD-L1. Alternatively, the assay involves detecting the expression of the immune checkpoint protein, preferably VISTA, in an immune cell and a cell expressing a second immune checkpoint protein, such as a cell expressing PD-L1. More preferably, the assay involves detecting the expression of the immune checkpoint protein, preferably VISTA, in a tumour cell, in an immune cell, and in an immune checkpoint-expressing cell.

Briefly, cells are labelled with binding antibodies specific for the immune checkpoint protein, e.g., VISTA, and: (1) at least one tumour cell marker; (2) at least one immune cell marker; and/or (3) at least one tumour cell marker and at least one immune cell marker. The binding antibodies are then visualised in the tumour samples by generating at least three distinct detectable signals: a first detectable signal that correlates with the location of antibody binding to the the immune checkpoint protein, such as VISTA; a second detectable signal that correlates with the location of the tumour cell-specific binding antibody; and a third detectable signal that correlates with the location of the immune cell marker. Of course, the skilled person will easily realise that, when more than one type of immune cells is to be detected (e.g., cytotoxic T cells, M2 macrophages, MDSCs, etc.), binding antibodies specific for each of these immune cell types will be used; accordingly, each of these binding antibodies will be visualised by generating an appropriate distinct detectable signal. Each of the detectable signals is distinguishable from one another.

The present disclosure thus relates to a multiplex method of scoring a tumour tissue sample stained with the immune checkpoint protein, preferably VISTA, comprising:
- contacting the tissue sample with an primary antibody directed to the the immune checkpoint protein, (e.g., VISTA); and
- contacting the same tissue sample with plurality of antibodies, the plurality of antibodies comprising:
   - at least one antibody directed to a tumour cell-specific marker; and/or
   - at least one antibody directed to an immune cell-specific marker;
- visualising each of the antibodies in the tissue sample with a reagent that generates a detectable signal corresponding to each of the antibodies, wherein the antibody against the immune checkpoint protein, preferably VISTA, has a first detectable signal, the antibody directed to the tumour cell-specific marker has a second detectable signal distinguishable from the first detectable signal, and the antibody directed to an immune cell- specific marker has a third detectable signal distinguishable from the first detectable signal and the second detectable signal;
- quantifying the number of cells expressing the immune checkpoint protein, preferably VISTA, for each cell type; and
- scoring the tumour based on this quantification.

Preferably, the plurality of antibodies comprises an antibody directed to a tumour cell-specific marker, an antibody directed to a cytotoxic T cell-specific marker, an antibody directed to a marker specific for myeloid-derived cells (MDSCs), an antibody directed to an M2 macrophage-specific marker, and an antibody specific for cells expressing a second immune checkpoint protein. Accordingly, each antibody can be visualised in the tissue sample with a reagent that generates a detectable signal corresponding to each of the antibodies, and which is distinguishable from the signal of the other reagents.

Optionally, a counterstain may be provided in an extra detectable signal.

### Tumour Cell Markers

Any marker capable of distinguishing tumour cells from non-tumour cells may be used. Examples of tumour cell-specific biomarkers may include but are not limited to: cytokeratins detectable with the pan keratin antibody (e.g., basic cytokeratins, many of the acidic cytokeratins), other cytokeratins such as cytokeratin 7 (CK7) and cytokeratin 20 (CK20), chromogranin, synaptophysin, CD56, thyroid transcription factor-1 (TTF-1), p53, leukocyte common antigen (LCA), vimentin, smooth muscle actin, or the like.

### Immune Cell Markers

Any marker capable of distinguishing immune cells from non-immune cells may be used. By way of non-limiting example, immune-populations of cells of relevance for identification in a clinical setting include CD4+ T cells including regulatory T cells, CD8+ T cells (cytotoxic), B cells (including naive, activated, memory and plasma cells), monocytes, dendritic cells, MDSCs, and macrophages (preferably M2). Preferably, the immune cell is selected in the group consisting of cytotoxic T cells (CD8+ T cells), M2 macrophages, and MDSCs.

Examples of immune cell-specific biomarkers may include but are not limited to: CD3, CD4, CD8, CD19, CD20, CD11c, CD14, CD33, CD56, CD66b, CD68, CD123, CD163, and CD206. Preferably, different immune cell markers are used, thereby allowing to distinguish several immune cell types. For example, a specific immune cell marker is used for each of cytotoxic T cells, M2 macrophages, myeloid-derived cells (MDSCs), etc.

In one example, a lymphocyte-specific marker is used. For example, a cytotoxic T-cell specific marker, such as CD3, CD4, or CD8, or a B-cell specific marker, such as CD19 or CD20 may be used. In a specific embodiment, the cytotoxic T-cell cell marker is CD8.

In one example, a M2 macrophage marker is used, such as e.g., CD68, CD123, or CD206. In a specific example, the M2 macrophage marker is CD123.

In another example, a MDSC-specific marker is used. Preferably, the MDSC-specific marker is CD33.

### Immune checkpoint markers

Identifying tumours comprising cells expressing a first immune checkpoint protein, such as VISTA, and cells expressing another immune checkpoint is particularly useful for designing an efficient immunotherapy. Any immune checkpoint proteins may thus be used in the present assays. Preferably, the immune checkpoint protein is CTLA-4, PDL1, PDL2, PD1, B7-H3, B7-H4, BTLA, HVEM, TIGIT, TIM3, GAL9, LAG3, KIR, 2B4, CD160 (also referred to as BY55), CGEN-15049, CHK1 and CHK2 kinases, IDO1, A2aR, or any one of various B7 family ligands.. More preferably, the immune checkpoint is PD-1 or PD-L1. Even more preferably, the immune checkpoint inhibitor is PD-L1.

### Tumour Samples

The present methods are compatible with tumour samples suitable for histochemical or cytochemical analysis, including, for example, fresh frozen, formalin-fixed paraffin-embedded (FFPE) samples, cytological smears (such as cervical smears), isolates of circulating tumour cells, etc. In a specific aspect, the sample is a FFPE sample of tumour tissue. Preferably, the sample used in the methods described herein comprises both tumour cells and cells of the TME.

The tumour may be any type of solid tumour. In some instances, the tumour includes Adrenocortical Carcinoma; Adrenocortical Carcinoma, Childhood; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumour, Adult; Brain Tumour, Brain Stem Glioma, Childhood; Brain Tumour, Cerebellar Astrocytoma, Childhood; Brain Tumour, Cerebral Astrocytoma/Malignant Glioma, Childhood; Brain Tumour, Ependymoma, Childhood; Brain Tumour, Medulloblastoma, Childhood; Brain Tumour, Supratentorial Primitive Neuroectodermal Tumours, Childhood; Brain Tumour, Visual Pathway and Hypothalamic Glioma, Childhood; Brain Tumour, Childhood (Other); Breast Cancer; Breast Cancer and Pregnancy; Breast Cancer, Childhood; Breast Cancer, Male; Bronchial Adenomas/Carcinoids, Childhood: Carcinoid Tumour, Childhood; Carcinoid Tumour, Gastrointestinal; Carcinoma, Adrenocortical; Carcinoma, Islet Cell; Carcinoma of Unknown Primary; Primary; Cerebellar Astrocytoma, Childhood; Cerebral Astrocytoma/Malignant Glioma, Childhood; Cervical Cancer; Childhood Cancers; Clear Cell Sarcoma of Tendon Sheaths; Colon Cancer; Colourectal Cancer, Endometrial Cancer; Ependymoma, Childhood; Epithelial Cancer, Ovarian; Oesophageal Cancer; Oesophageal Cancer, Childhood; Ewing's Family of Tumours; Extracranial Germ Cell Tumour, Childhood; Extragonadal Germ Cell Tumour; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumour; Germ Cell Tumour, Extracranial, Childhood; Germ Cell Tumour, Extragonadal; Germ Cell Tumour, Ovarian; Gestational Trophoblastic Tumour; Glioma. Childhood Brain Stem; Glioma. Childhood Visual Pathway and Hypothalamic; Head and Neck Cancer; Hepatocellular (Liver) Cancer, Adult (Primary); Hepatocellular (Liver) Cancer, Childhood (Primary); Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Lip and Oral Cavity Cancer; Liver Cancer, Adult (Primary); Liver Cancer, Childhood (Primary); Lung Cancer, Non-Small Cell; Lung Cancer, Small Cell; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumour; Ovarian Low Malignant Potential Tumour; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumours, Childhood; Pituitary Tumour; Plasma Cell Neoplasm/Multiple Myeloma; Pleuropulmonary Blastoma; Pregnancy and Breast Cancer; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumours; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumours, Childhood; Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumour, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumour.

### Antibodies

The present methods preferably use antibodies or antigen-binding fragments thereof which specifically recognise a biomarker of interest. As used herein, the term "antibody" refers to any form of antibody that exhibits the desired biological or binding activity. Thus, it is used in the broadest sense and specifically covers, but is not limited to, monoclonal antibodies (including full length monoclonal antibodies), polyclonal antibodies, multispecific antibodies (e.g., bispecific antibodies), humanised antibodies, fully human antibodies, chimeric antibodies and camelised single domain antibodies.

As used herein, unless otherwise indicated, "antibody fragment" or "antigen binding fragment" refers to antigen binding fragments of antibodies, i.e., antibody fragments that retain the ability to bind specifically to the antigen bound by the full-length antibody, e.g., fragments that retain one or more CDR regions. Examples of antibody binding fragments include, but are not limited to, Fab, Fab', F(ab')2, and Fv fragments; diabodies; linear antibodies; single-chain antibody molecules, e.g., sc-Fv; nanobodies and multispecific antibodies formed from antibody fragments.

Antibodies recognising specifically can be obtained commercially (see e.g., the example). Alternatively, antibodies can be obtained by any method known to the skilled person. For example, monoclonal antibodies of interest can be obtained from hybridomas. Methods for generating hybridomas are known in the art (*see*, *e.g.*, Kohler and Milstein, 1975, Nature 256:495). Generally, a host animal, such as a mouse is immunised with an immunogen, such as a peptide of interest, to elicit the development of lymphocytes, for example spleen cells, that produce antibodies capable of specifically binding the immunogen. Alternatively, isolated lymphocytes, including spleen cells, lymph node cells, or peripheral blood lymphocytes, can be immunized *in vitro.* Lymphocytes are then fused with an immortalized cell line, such as a myeloma cell line, using a suitable fusing agent (e.g., polyethylene glycol), to form a hybridoma cell line. Suitable immortalised cell lines can be of mammalian origin, such as murine, bovine, or human. Hybridoma cells are then cultured in any suitable medium that contains one or more substances that inhibit growth or survival of the unfused, immortalised cells.

The antibodies used in the methods disclosed herein are preferably labelled antibodies. The antibodies can be used diagnostically, for example, to detect expression of a target of interest in specific cells, tissues, or serum; or to monitor the development or progression of an immunologic response as part of a clinical testing procedure to, *e.g.*, determine the efficacy of a given treatment regimen. Detection can be facilitated by coupling the antibody to a detectable substance or "label." A label can be conjugated directly or indirectly to an antibody of the disclosure. The label can itself be detectable (*e.g*., radioisotope labels, isotopic labels, or fluorescent labels) or, in the case of an enzymatic label, can catalyse chemical alteration of a substrate compound or composition which is detectable. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance can be coupled or conjugated either directly to the antibody (or fragment thereof) or indirectly, through an intermediate (such as, for example, a linker known in the art) using techniques known in the art.

Examples of suitable fluorescent materials include, for example, the Opal^{®} reagents, available from Akoya Biosciences (Menlo Park, CA), Brilliant^{™} Ultraviolet 395 (BUV395), Brilliant^{™} Violet 480 (BV480), Brilliant Violet 421^{™}, Brilliant^{™} Violet 421 (BV421), Brilliant^{™} Violet 510 (BV510), Brilliant Violet 570^{™}, Brilliant Violet 605^{™}, Brilliant Violet 650^{™}, Brilliant Violet 711^{™}, BD Horizon^{™}, BD Horizon^{™} V500, Brilliant^{™} Blue 515 (BB515), Fluorescein Isothiocyanate (FITC), Alexa Fluor 488 (AF488), Alexa Fluor 532 (AF532), R-phycoerythrin (PE), Alexa Fluor 594 (AF594), PE-Dazzle 594 (PE594) or PE-CF594 (CF594), Alexa Fluor 647 (AF647), Allophycocyanin (APC), BD Horizon^{™} 700 (BB700), Alexa Fluor 700 (AF700), APC/Alexa Fluor 750, APC/Fire 750, APC-R700, APC-Cy and AF750.

### Visualisation of biomarkers

As previously described, the multiplex assays described herein feature staining of VISTA as well as staining of one or more of tumour and immune cell markers to generate a detectable signal that correlates with the location at which an exogenous binding entity has bound to the sample.

Multiplexed imaging techniques, including multiplexed immunofluorescent (mIF) imaging, enable the analysis of many target biomarkers in a single tissue sample. In a single imaging step, up to 8 targets can be simultaneously measured using a combination of suitably chosen stains and computational processing methods such as spectral unmixing to separate measured fluorescent signals corresponding to the different stains. Suitable stains include in particular the Opal^{®} reagents, available from Akoya Biosciences (Menlo Park, CA). In particular, new Opal^{®} IHC technique allows multiplexed staining of tissues up to seven to nine colours. Alternately, suitable stains include the UltiMapper I/O PD-L1 kit, the UltiMapper I/O Immuno8 kit, or InSituPlex stains (available from Ultiview Inc., Cambridge MA).

Multiplexed staining, imaging and spectral unmixing methods are described for example in the following U.S. patents and patent application publications: U.S. Patents Nos. 7,555,155; 8,462,981; 8,330,087; 9,541,504; and 10,126,242; and U.S. patent application publication No. US 2019/0339203.

Sequential imaging cycles can also be performed to further increase the number of target analytes that can be interrogated. For example, following each cycle of staining and imaging, stains applied to the sample can be removed or inactivated, and a new cycle of staining and imaging can be initiated in which a new set of stains is introduced, and one or more images of the newly introduced stains are obtained. Such methods can be particularly useful for assays that investigate a large number of target analytes. One example of such an assay is an antigen-targeting assay that targets a panel of tumour-related markers in a tissue sample.

Suitable reagents for performing sequential imaging cycles include, for example, the CODEX^{®} reagents available from Akoya Biosciences. Aspects of multi-cycle labelling and imaging of target analytes in biological samples are described for example in the following patents and patent publications: U.S. Patents Nos. 9,909,167 and 10,370,698; PCT applications Nos. WO 2021/067475; WO 2020/163397; and WO 2021/127637.

As previously described, the assays of the present invention feature staining of VISTA as well as staining of one or more differentiating markers. Methods of staining may include immunohistochemistry (IHC). Staining techniques may be performed on various biological samples, such as tissue (e.g., fresh frozen, formalin-fixed paraffin-embedded (FFPE)) and cytological samples.

Preferably, the present method comprises performing a multiplexed staining/labelling and imaging assay. Such assays can include, for example, exposing the sample to probes that target one or more different biomarkers in the sample, and imaging the sample to obtain information about the localising of the probes.

A variety of different types of probes can be introduced. Preferably, the probes target specific types of antigens in the sample, and consist of antibodies that are linked directly or indirectly to fluorescent moieties (e.g., "labels"). Each type of antibody can be linked to a different, spectrally distinguishable label, so that multispectral images of the sample reveal the locations of each of the different types of antibodies - and therefore their specific target antigens - in the sample. Multiple cycles of imaging and staining/labelling can optionally be performed, with optional inactivation/quenching of the previous cycle's labels before a new set of probes is introduced in the next cycle.

In certain embodiments, the probes consist of antibodies that are linked to oligonucleotides, such that each type of antibody is linked directly or indirectly to a different type of oligonucleotide. All such probes can be introduced in a single sample labelling step, or in successive labelling steps. After probes have bound to the target antigens in the sample, labelling agents are introduced. Each type of labelling agent includes an oligonucleotide that is complementary to the oligonucleotide of one of the probes, and a fluorescent moiety linked directly or indirectly to the oligonucleotide of the labelling agent. The oligonucleotides of the probe and labelling agent hybridise, localising the labelling agent in the sample where the target analyte is located. When the sample is imaged, the locations of the labelling agents reveal the locations of the corresponding analytes. Multiple cycles of labelling and imaging can be performed. Between cycles, labelling agents from the prior art are generally removed via dehybridisation from their corresponding probes prior to introduction of a new set of labelling agents.

In some embodiments, the probes consist of antibodies or oligonucleotides that are linked to a catalytic agent such as horseradish peroxidase (HRP). The example is exposed to probes which selectively bind to one of the analytes (e.g., antigens, proteins, RNA), localising the catalytic agent in the sample with the analyte. The sample is then exposed to a tyramide-linked labelling agent. The catalytic agent catalyses activation of the tyramide and deposition of the labelling agent in the sample in proximity to the target analyte. The deposited labelling agent is imaged (e.g., by measuring fluorescence emission), which identifies the target analyte in the sample. The deposited labelling agent can then be removed or inactivated, and another cycle of labelling and imaging performed to identify another target analyte.

A non-limiting example of a detailed protocol is described in Example 1 below. Briefly, samples of interest are stained for the first immune checkpoint protein, preferably VISTA. The sample is incubated first with a probe comprising an anti-VISTA primary antibody. The primary antibody against the first immune checkpoint protein (e.g., VISTA)is detected with a first colour. In Example 1, the sample is incubated with an OPAL 620-conjugated antibody against the first immune checkpoint protein (e.g., VISTA), producing the first colour.

The sample is then stained for a first differentiating marker, e.g., a marker that is tumour cell-specific. Examples of tumour cell-specific biomarkers may include but are not limited to: cytokeratins detectable with the pan keratin antibody (e.g., basic cytokeratins, many of the acidic cytokeratins), other cytokeratins such as cytokeratin 7 (CK7) and cytokeratin 20 (CK20), chromogranin, synaptophysin, CD56, thyroid transcription factor-1 (TTF-1), p53, leukocyte common antigen (LCA), vimentin, smooth muscle actin, or the like. The skilled person will easily substitute another appropriate tumour cell-specific biomarker for the cytokeratins as described in Example 1. The sample is incubated first with a primary antibody (e.g., anti-pan keratin antibody) against the first differentiating marker (tumour cell-specific marker). This antibody is conjugated to OPAL 520 which produces the second colour.

The sample is then stained for an immune cell-specific marker. In particular, the sample may be stained for a biomarker specific for cytotoxic T cells. Non-limiting examples of cytotoxic T cell-specific biomarkers include CD8 or any other CD marker. Cytotoxic T cell-specific biomarkers are well known to one of ordinary skill in the art. One of ordinary skill in the art could substitute another appropriate cytotoxic T cell-specific biomarker for CD8 as described in Example 1. The sample is incubated first with a primary antibody (e.g., anti-CD8 antibody) against the third differentiating marker (cytotoxic T cell-specific marker). The anti-differentiating marker primary antibody is conjugated with OPAL 570 which produces a third colour.

The sample is then stained for another immune cell-specific marker, such as an M2 macrophage-specific biomarker. Non-limiting examples of M2 macrophage-specific biomarkers include CD163. M2 macrophage-specific biomarkers are well known to one of ordinary skill in the art. The skilled person could substitute another appropriate M2 macrophage-specific biomarker for CD163 as described in Example 1. The sample is incubated first with a primary antibody (e.g., anti-CD163 antibody) against the fourth differentiating marker (M2 macrophage-specific marker). The anti-differentiating marker primary antibody is conjugated with OPAL 540 which produces a fourth colour.

The sample is then stained for another immune cell-specific marker, such as a biomarker specific for the myeloid lineage, thereby allowing staining of the MDSCs in the TME. Non-limiting examples of myeloid lineage-specific biomarkers include CD33. Myeloid lineage-specific biomarkers are well known to one of ordinary skill in the art. The skilled person could substitute another appropriate myeloid lineage-specific biomarker for CD33 as described in Example 1. The sample is incubated first with a primary antibody (e.g., anti-CD33 antibody) against the fifth differentiating marker (myeloid lineage-specific marker). The anti-differentiating marker primary antibody is conjugated with OPAL 650 which produces a fifth colour.

The sample is then stained for a marker specific of cells expressing a second immune checkpoint protein. Non-limiting examples of biomarkers specific for such cells include PD-L1. Cells expressing a second immune checkpoint protein and biomarkers thereof are well known to the skilled person. The skilled person could substitute another appropriate - biomarker specific of cells expressing a second immune checkpoint protein for PD-L1 as described in Example 1. The sample is incubated first with a primary antibody (e.g., anti-PD-L1 antibody) against the sixth differentiating marker (marker specific for cells expressing a second immune checkpoint protein). The anti-differentiating marker primary antibody is conjugated with OPAL 690 which produces a sixth colour.

In some embodiments, the samples are then counterstained, producing a seventh colour (the seventh colour being different from the first, second, third, fourth, fifth, and sixth colours). In some embodiments, the counterstain comprises haematoxylin; however, the counterstain is not limited to haematoxylin. Alternative counterstains are well known to one of ordinary skill in the art. For example, the counterstain may comprise methylene blue, nuclear red, toluidine blue, eosin, methyl green, or the like. The particular counterstain is generally selected to produce contrast so as to enhance visibility.

The present method is not limited to staining in any particular order. For example, Example 1 describes staining first for the first immune checkpoint protein (e.g., VISTA), then staining for the tumour cell-specific marker, then staining for the cytotoxic T cell-specific marker, then staining for the M2 macrophage-specific marker, etc. However, in some examples, the tumour cell-specific marker (or cytotoxic T cell-specific marker or M2 macrophage-specific marker) is stained first, followed by the staining for the first immune checkpoint (e.g., VISTA), etc.

Following the staining procedure, one or more images of the sample that include spectral contributions from the various markers are obtained with an imaging system, e.g., the Vectra^{®} 3 automated quantitative pathology imaging system (Akoya Biosciences Inc., Menlo Park CA).

In the present case, a first image of the biological sample is generated by measuring the fluorescence emitted by the labelled antibody against the first immune checkpoint protein (e.g., a labelled anti-VISTA antibody). The generated first image corresponds to a pattern of VISTA staining in the biological sample. Likewise, a second image of the biological sample is generated, that corresponds to a pattern of the tumour cell-specific marker in the biological sample; a third image of the biological sample is generated, that corresponds to a pattern of the cytotoxic T cell marker staining in the biological sample; a fourth image of the biological sample is generated, that corresponds to a pattern of the M2 macrophage marker staining in the biological sample; a fifth image of the biological sample is generated, that corresponds to a pattern of myeloid-derived cell marker staining in the biological sample; and a sixth image of the biological sample is generated, that corresponds to a pattern of the second immune checkpoint marker staining in the biological sample.

These images can be used to generate, and optionally display and/or store, a simulated image of the sample (see e.g., WO 2022/020340). The image can be viewed, annotated, and used as described herein to take further action, such as to assess whether the sample should be further analysed in a multispectral staining/labelling and imaging protocol. The image can also be used to identify particular regions of interest in a sample, which are then analysed computationally following the multispectral staining/labelling and imaging protocol.

In some instances, the simulated image is generated based on both a fluorescent image and a transmitted-light brightfield image of the sample. The brightfield image of the sample can be registered with the fluorescent image to create a simulated image that incorporates information about absorptive features in the sample.

The methods and systems described herein can implement workflows in which multispectral images of the sample are analysed together with the simulated image. For example, both the simulated image and the multispectral sample images can be displayed to a user (e.g., side-by-side, overlaid, or alternating between these images), along with quantitative metrics derived from the images such as cell locations, density maps, and co-located target analytes.

Once the image is generated, the samples are then interpreted and scored (scoring is described below). By way of example, in the case of an assay that stains for VISTA (or any other example of a first immune checkpoint protein), a tumour cell-specific marker (e.g., cytokeratins), and an immune cell-specific marker (e.g., CD8 for cytotoxic T cells), the cells that score for the first colour and third colour but not the second colour are VISTA positive immune cells, cells that score for both the first colour and the second colour (but not the third colour) are VISTA positive cancer cells, and cells that score for the second colour but not the first colour nor the third colour are VISTA negative cancer cells. The same method can be applied to up to the six colours described herein, leading to the identification (and optionally quantification) of cells expressing VISTA in each of the cell types assayed. In some embodiments, the first colour and second colour overlap (or others overlap), producing an eighth (different) colour. This overlap colour may help scoring. In some instances, the intensity of the signal of each colour is measured.

In some embodiments, the staining (e.g., the first immune checkpoint protein, for example VISTA, and the differentiating marker) occurs sequentially. In some embodiments, the staining occurs simultaneously.

### Scoring

Samples are then scored. The second, third, fourth, fifth, or sixth colour directs scoring of the first immune checkpoint protein (for example VISTA) in either of the cell types described above. The use of at least another colour may improve scoring. For example, the use of the third colour may help to clarify which cell type (tumour vs. cytotoxic T cells) is positive for the first immune checkpoint protein (e.g., VISTA). This can help improve the accuracy of the calculation of the number of VISTA positive cytotoxic T cells, tumour cells negative for the first immune checkpoint protein (e.g., VISTA), and tumour cells positive for the first immune checkpoint protein (e.g., VISTA). The skilled person will easily realise that using at least 2, at least 3, at least 4, at least 5 colours in addition to the first colour will improve the accuracy of the scoring. The simulated image and the multispectral images described above are particularly useful for scoring accurately the samples.

A positive result (e.g., a result positive for the first immune checkpoint protein, e.g., VISTA) may be calculated in a variety of ways and is not limited to the examples described herein.

In some examples, the number of VISTA positive cells in a specific cell type (e.g., tumour cells, cytotoxic T cells, M2 macrophages, MDSCs, or checkpoint protein-expressing cells) or the area of the first immune checkpoint protein (e.g., VISTA)that is associated with this particular cell type (e.g., the area of the slide covered with the first immune checkpoint protein (e.g., VISTA) due to cells of that cell type) or the percentage of cells positive for the first immune checkpoint protein (e.g., VISTA) in this cell type may be calculated and then factored into an equation to calculate a score. For example, if the score is above a threshold for positivity for the first immune checkpoint protein (e.g., VISTA) then the sample is positive for the first immune checkpoint protein (e.g., VISTA), and if the score is below the threshold for positivity for the first immune checkpoint protein (e.g., VISTA) then the sample is negative for the first immune checkpoint protein (e.g., VISTA).

Non-limiting examples of scoring calculations are briefly described below:
In some examples, a positive result is determined by calculating the percentage of cells positive for the first immune checkpoint protein (e.g., VISTA) in a specific cell type and determining if that percentage is above the threshold for positivity or a predetermined cut-off. For example, in some embodiments, the minimum percentage of cells expressing the first immune checkpoint protein (e.g., VISTA) that confers positivity for the first immune checkpoint protein (e.g., VISTA), e.g., 5% or more cells positive for the first immune checkpoint protein (e.g., VISTA) in the cell type of interest confers positivity for the first immune checkpoint protein (e.g., VISTA), 10% or more cells positive for the first immune checkpoint protein (e.g., VISTA) in the cell type of interest confers positivity for the first immune checkpoint protein (e.g., VISTA), 25% or more cells positive for the first immune checkpoint protein (e.g., VISTA) in the cell type of interest confers positivity for the first immune checkpoint protein (e.g., VISTA), 50% or more cells positive for the first immune checkpoint protein (e.g., VISTA) in the cell type of interest confers positivity for the first immune checkpoint protein (e.g., VISTA), etc.

In some examples, a positive result is determined by calculating the number of cells positive for the first immune checkpoint protein (e.g., VISTA) in a particular cell type divided by the total number of cells (e.g., number of tumour cells plus cytotoxic T cells plus M2 macrophages plus MDSCs plus checkpoint protein-expressing cells) and determining if that value is above the threshold for positivity (e.g., value greater than 0.15, value greater than 0.25, value greater than 0.5, etc.).

In some examples, a positive result is determined by calculating the sum of the percentage of cells positive for the first immune checkpoint protein (e.g., VISTA) in all the assayed cell types and determining if that value is above the threshold for positivity (e.g., value greater than 40, value greater than 50, value greater than 60, etc.).

In some examples, a positive result is determined by calculating the number of cells positive for the first immune checkpoint protein (e.g., VISTA) in a particular cell type divided by the number of cells negative for the first immune checkpoint protein (e.g., VISTA) in that particular cell type and determining if that value is above the threshold for positivity (e.g., value greater than 1.5, value greater than 1.8, value greater than 2, etc.).

As discussed above, positivity for the first immune checkpoint protein (e.g., VISTA) may be determined by calculating the percentage of cells positive for the first immune checkpoint protein (e.g., VISTA) in each of the cell types assayed. For example, staining in greater than about 1% of cells (e.g., tumour cells, cytotoxic T cells, M2 macrophages, MDSCs, or second-checkpoint protein-expressing cells) is scored as positive for the first immune checkpoint protein (e.g., VISTA). In another example, staining in greater than about 5% of cells (e.g., t tumour cells, cytotoxic T cells, M2 macrophages, MDSCs, or second-checkpoint protein-expressing cells) is scored as positive for the first immune checkpoint protein (e.g., VISTA). In some embodiments, staining in greater than about 10% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 15% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 20% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 25% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 30% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 35% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 40% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 45% of cells (e.g., tumour cells, cytotoxic T cells, M2 macrophages, MDSCs, or second-checkpoint protein-expressing cells) is scored as positive for the first immune checkpoint protein (e.g., VISTA). In some embodiments, staining in greater than about 50% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 55% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 60% of cells (e.g., tumour cells) is scored as VISTA positive. In some examples, staining in greater than about 65% of cells (e.g., tumour cells, cytotoxic T cells, M2 macrophages, MDSCs, or checkpoint protein-expressing cells) is scored as positive for the first immune checkpoint protein (e.g., VISTA). In some examples, staining in greater than about 70% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 75% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 80% of cells (e.g., tumour cells, cytotoxic T cells, M2 macrophages, MDSCs, or second-checkpoint protein-expressing cells) is scored as positive for the first immune checkpoint protein (e.g., VISTA). In some examples, staining in greater than about 90% of cells (e.g., tumour cells, cytotoxic T cells, M2 macrophages, MDSCs, or second-checkpoint protein-expressing cells) is scored as positive for the first immune checkpoint protein (e.g., VISTA).

The positivity of the sample may also be determined by the degree or intensity of staining (e.g., heavy staining may be positive and light staining may be negative). In some embodiments, scoring methods may feature scoring samples on an intensity scale, e.g., of 0 to 3, for expression of the first immune checkpoint protein (e.g., VISTA) (as described, for example, in WO 2020/016459A1). In some embodiments, samples are scored based on intensity and percentages of cells staining. For example, H scores are calculated as (see e.g., Ram et al. PLoS One. 16(9):e0245638 (2021)): 1 * (percentage of tumour cells staining at 1+ intensity) + 2 * (percentage of tumour cells staining at 2+ intensity) + 3 * (percentage of cells staining at 3+ intensity) = H score (a value between 0 and 300). Other scoring methods have been described and are well known to one of ordinary skill in the art.

### Applications

### Diagnosis methods

The methods described herein are notably useful for diagnosing a patient with a disease. In some cases, the presence or absence of the first immune checkpoint protein (e.g., VISTA)in a particular cell type (e.g., MDSCs) in the patient's sample can indicate that the patient has a particular disease (e.g., cancer). In some cases, a patient can be diagnosed with a disease by comparing a sample from the patient with a sample from a healthy control. In this example, a level of a biomarker, relative to the control, can be measured. A difference in the level of a biomarker in the patient's sample relative to the control can be indicative of disease. In some cases, one or more biomarkers are analysed in order to diagnose a patient with a disease. The compositions and methods of the disclosure are particularly suited for identifying the presence or absence of, or determining expression levels, of a plurality of biomarkers in a sample.

In another aspect, the present methods are used for ranking a plurality of tissue samples. For instance, the tissue samples can be from different patients. According to this specific instance, the samples can be from the same tissue or from different tissues. Alternatively, the tissue samples are from the same patient. In the case, the samples are from different tissues.

According to this specific aspect, the disclosure relates to a method for ranking a plurality of tissue samples, comprising the steps of:
a) scoring each of the tissue samples using the scoring method described herein, and
b) ranking the tissue samples based on the scoring of a).

In another aspect, the methods described herein are also particularly useful for identifying a patient sub-group within a group of patients. Notably, these methods are particularly useful for identifying patients expressing for the first immune checkpoint protein (e.g., VISTA) on their tumour cells or in the TME within a group of cancer patients.

In another aspect the disclosure relates to a method of identifying a patient subgroup from within a group of patients comprising:
a) scoring a tumour tissue sample from each of the patients of the group of patients using the scoring method described herein, and
b) determining that a patient is in a sub-group based on the scoring of a).

In one embodiment the patient sub-group is a group of patients having a shared cellular phenotype.

In one embodiment the patient sub-group is defined by the presence of one or more target antigens, biomarkers and/or cell types.

For example, a patient sub-group can be defined by the same pattern of co-localisation of the first immune checkpoint protein (e.g., VISTA) and one or more biomarkers.

### Methods of treatment

In some cases, the methods herein can be used to determine a treatment plan for a patient. The presence or absence of for the first immune checkpoint protein (e.g., VISTA)in a particular cell type may indicate that a patient is responsive to or refractory to a particular therapy. For example, a presence of for the first immune checkpoint protein (e.g., VISTA)on immune cells such as cytotoxic T cells and MDSCs may indicate that a cancer is refractory to a specific therapy, e.g., a chemotherapy, and an alternative therapy can be administered. In some cases, a patient is currently receiving the therapy and the presence or absence of one or more biomarkers may indicate that the therapy is no longer effective.

Accordingly, the present disclosure also provides a method for predicting a treatment response of a patient to a proposed treatment for a cancer comprising:
a) scoring a patient's tumour tissue sample using the scoring method described herein, and
b) determining that the patient will or will not be responsive to the proposed treatment based on the score of the tumour tissue sample, optionally in comparison to a suitable control.

A patient is notably be determined by the present method to be responsive to the treatment when the patient's tumour tissue sample is positive for the first immune checkpoint protein (e.g., VISTA) for at least one of tumour cells, cytotoxic T cells, M2 macrophages, and MDSCs. The more cell types are positive for the first immune checkpoint protein (e.g., VISTA), the more likely the patient is to respond to the treatment.

Specifically contemplated herein as embodiments of this method for predicting a treatment response of a cancer patient are all of the embodiments set forth herein that relate to the previous aspects of the invention set forth herein, including but not limited to antibodies, fluorophores, Ab-FPs, target antigens, biomarkers, tissues, cell types, and methods of detecting a plurality of target antigens, biomarkers and cell types including labelling, multispectral imaging and analysis of multispectral images, but not limited thereto.

In a specific instance, the treatment for cancer comprises the administration of an therapeutic agent directed against the first immune checkpoint protein. Preferably, this anti-therapeutic agent is anti-VISTA therapeutic agent. For example, the anti-VISTA therapeutic agent can be an antibody which inhibits VISTA checkpoint inhibitor function. Antibodies inhibiting VISTA function which induce strong tumor growth inhibition have been previously described and are particularly useful for treating cancer (see WO 2014/197849, WO 2016/094837, WO 2022/229469, and WO 2023/213814). Other anti-VISTA antibodies with anti-cancer properties have also been described in the art (see e.g., WO 2014/039983A1, WO 2015/145360A1, WO 2015/097536, WO 2017/137830, WO 2017/181139).

A monoclonal antibody 26A capable of inhibiting VISTA immune suppression, thereby enhancing antitumour immune response, is described in WO 2016/094837 and WO 2022/229469. This inhibition of VISTA immune suppression surprisingly requires the effector functions of 26A. Moreover, this antibody is capable of blocking the interaction between VISTA and each of its two binding partners, PSG-L1 and VSIG3, said interaction resulting in inhibition of T cell function. Accordingly, the antibody inhibits tumour proliferation in vivo. Surprisingly, this inhibition of tumour proliferation requires the effector functions of the antibody.

The CDRS of the antibody 26A are displayed below:
- GFSFTGYT (SEQ ID NO:3)
- ISPYDGGT (SEQ ID NO:4)
- ARRAYGYAMDY (SEQ ID NO:5)
- SSVSY (SEQ ID NO:6)
- DTS (SEQ ID NO:7)
- QQWSSYPFT (SEQ ID NO:8)

The V_{H} and V_{L} of antibody 26A are represented by SEQ ID NO:9 and SEQ ID NO:10, respectively. The complete heavy chain of 26A has the sequence represented by SEQ ID NO:11 and the complete light chain of 26A has the sequence represented by SEQ ID NO:12.

In a particular instance of the method for predicting a treatment response of a cancer patient, when the patient's tumour tissue sample is positive for the first immune checkpoint protein (e.g., VISTA) for at least one of tumour cells, cytotoxic T cells, M2 macrophages, and MDSCs, and the same sample is positive for PD-L1, the treatment comprises the administration of the anti-VISTA monoclonal antibody and of an anti-PD-1 antagonist. Preferably, the anti-PD-1 antagonist is an antagonistic monoclonal antibody.

Expression of immune checkpoint proteins such as VISTA has been shown to be upregulated after administration of some systemic therapies, which could lead to adaptive resistance (Martinet al. Front Immunol. 14:1086102 2023)).

In another aspect, the disclosure relates to a method of prognosing an anticancer therapy in patient comprising:
a) taking a first tumour tissue sample from the patient at a first time point;
b) taking a second tumour tissue sample from the patient at a second time point;
c) scoring the first tumour sample from patient using the scoring method described herein, thereby generating a first score;
d) scoring the second tumour sample from the patient using the scoring method described herein, thereby generating a second score;
e) comparing the first score of c) and the second score of d), and
f) determining the prognosis of the patient based on the comparison of e).

In a first instance, the prognosis of the patient is bad when the second score of d) is higher than the first score of c). In another instance, the prognosis of the patient is good when the first score of c) is higher than the second score of d).

The anticancer therapy can be any type of therapy against cancer known to the skilled person.

For example, the anticancer therapy can be an immune checkpoint inhibitor. Exemplary immune checkpoint inhibitors include anti-CTLA-4 antibody (*e.g.*, ipilimumab), anti-LAG-3 antibody (*e.g*., BMS-986016), anti-B7-H3 antibody, anti-B7-H4 antibody, anti-Tim3 antibody (*e.g.*, TSR-022, MBG453), anti-BTLA antibody, anti-KIR antibody, anti-A2aR antibody, anti CD200 antibody, anti-PD-1 antibody (e.g., pembrolizumab, nivolumab, cemiplimab, pidilizumab), anti-PD-L1 antibody (e.g., atezolizumab, avelumab, durvalumab, BMS 936559), anti-TIGIT antibody (e.g., tiragolumab, vibostolimab), anti-VSIG4 antibody, anti-CD28 antibody, anti-CD80 or - CD86 antibody, anti-B7RP1 antibody, anti-B7-H3 antibody, anti-HVEM antibody, anti-CD137 antibody *(e.g.,* urelumab), anti-CD137L antibody, anti-OX40 *(e.g.,* 9B12, PF-04518600, MEDI6469), anti-OX40L antibody, anti-CD40 or -CD40L antibody, anti-GAL9 antibody, anti-IL-10 antibody, fusion protein of the extracellular domain of a PD-1 ligand, *e.g.* PDL-1 or PD-L2, and IgG1 (*e.g.*, AMP-224), fusion protein of the extracellular domain of a OX40 ligand, *e.g.* OX40L, and IgG1 (*e.g.*, MEDI6383), IDO1 drug (e.g., epacadostat) and A2aR drug. A number of immune checkpoint inhibitors have been approved or are currently in clinical trials. Such inhibitors include ipilimumab, pembrolizumab, nivolumab, cemiplimab, pidilizumab, atezolizumab, avelumab, durvalumab, tiragolumab, vibostolimab, BMS 936559, urelumab, 9B12, PF-04518600, BMS-986016, TSR-022, MBG453, MEDI6469, MEDI6383, and epacadostat.

Alternatively, the anticancer therapy can be chemotherapy. Chemotherapeutic agents include for example docetaxel, paclitaxel, oxaliplatin, etoposide, 5-fluorouracil, gemcitabine, cisplatin, encorafenib, and binimetinib.

Hereinbelow, the present disclosure is explained in detail in view of the examples. However, the following examples are given only for exemplification of the present disclosure, and it is evident that the present disclosure is not limited to the following examples.

### EXAMPLE 1

### Purpose

V-domain Ig Suppressor of T-cell Activation (VISTA) is a B7 family member that acts as an immune checkpoint regulator. VISTA is expressed in various cell types such as tumour, myeloid, lymphoid, and endothelial cells. Its expression within the tumour microenvironment (TME) favours tumour immunosuppressive condition, immune check point resistance and is linked to poor prognosis in multiple tumour indications. Therefore, VISTA appeared as an interesting therapeutic target in oncology and encourage Pierre FABRE Laboratories to develop an Fc gamma human monoclonal antibody targeting VISTA, W0180 (K01401-020), that is currently being assessed in a Phase 1 clinical trial in patients with advanced solid tumours (NCT04564417). However, the variability of VISTA expression needs to be better documented to identify indications of interest that could increase the therapeutic response rate.

With the aim of targeting patients with high expression of VISTA in tumour for the development of this antibody, the expression of VISTA was investigated in different cell types such as tumour cells, cytotoxic T cells, myeloid cells, M2 macrophages, and PD-L1 positive cells, in 23 solid tumour indications gathering more than 1000 patients. Therefore, a 7-colour multiplex assay to quantify VISTA in combination with different immune and tumour cells markers was developed and validated.

In this assay, the Akoya Bio Opal Fluorophore detection system and Akoya Vectra 3 multispectral scanner were used. The Opal system uses Tyramide Signal Amplification technology (TSA) to detect low-intensity markers, and the Vectra 3 scanner uses multispectral technology to accurately separate the convoluted fluorescence images into the components from each fluorescence-labelled antibody (Parra et al., Sci Rep. 7(1):13380 (2017); Stack et al., Methods. 70(1):46-58 (2014)). For mIF image quantification, a semi-automated image analysis workflow combining conventional image analysis algorithms and machine learning is beneficial, especially when the number of images is large.

### Materials and Methods:

Multiplex immunofluorescent panel has been designed with validated antibodies to characterise VISTA positive cells on tumour cells (i.e., cytokeratin - CK), immune checkpoint markers (i.e., PD-L1,), infiltrating cytotoxic lymphocytes (i.e., CD8), M2 macrophages (- i.e., CD163) and myeloid-derived suppressor cells (MDSCs - i.e., CD33) as described in the table below:

**Table 1: Antibodies used in the study**

| **Markers** | **Vendor** | **Clone** | **Reference** | **IHC dilution** | **mIF dilution** |
|---|---|---|---|---|---|
| VISTA | Abcam | EPR21050 | Ab230950 | 1:500 | 1:500 |
| CD163 | Novus | EDHu-1 | NBP110-40686 | 1:500 | 1:500 |
| CD33 | Abcam | SP266 | ab199432 | 1:100 | 1:200 |
| PD-L1 | CST | E1L3N | 13684S | 1:100 | 1:50 |
| CD8 | CST | D8A8Y | 85336S | 1:50 | 1:100 |
| CK | Novus | AE-1 /AE-3 | NBP2- 29429 | 1:150 | 1:100 |

Each marker has been associated with fluorescent staining as described below.

**Table 2: Fluorescent labels used in the study**

| **HALO** | **Markers** | **Phenotype/definition** |
|---|---|---|
| OPAL 620 | **VISTA** | Cells expressing VISTA |
| OPAL 540 | **CD163** | Cells expressing CD163 (M2 macrophages) |
| OPAL 650 | **CD33** | Myeloid cells lineage |
| OPAL 690 | **PDL1** | Cells expressing PDL1 |
| OPAL 570 | **CD8** | Cytotoxic T cells |
| OPAL 520 | **CK** | Tumour cells / Epithelial cells |

Multiplex immunofluorescence has been validated to allow the detection of VISTA, CK, CD8, CD33, CD163 and PD-L1 positive cells:
Multiplex immunofluorescence (mIF) panel were performed on FFPE sample coming from Pierre Fabre's internal biobank gathering more than 1000 patients from 23 different solid tumour indications.

All samples were analysed in accordance with the Study Protocol, regulatory guidelines and QA/QC procedures delineated in Laboratory's Standard Operating Procedures ("SOPs").

Briefly Human FFPE samples were freshly sectioned (4µm) and processed to glass slides before staining steps. Prior to staining, all tissue slides were deparaffinized on the Leica Bond Rx autostainer (Leica Microsystems, Milton Keynes, UK) by baking for 30 min at 60 °C in BOND Dewax Solution at 72 °C and then rehydrating in ethanol.

After staining, FFPE slides were digitally imaged using the Vectra 3^{®} scanner platform (Akoya Biosciences Inc., Menlo Park CA) in their entirety using a 20x 0.75 NA objective to obtain an 0.4972 µm/pixel image resolution.

For each slide, the Vectra 3^{®} scanner platform provides mIF images which were analysed with the HALO platform (Indicalab, Albuquerque, NM). HALO is a pathology digital platform for cell-based image analysis that can separate up to five stains simultaneously within any cellular compartment. It is particularly useful for characterising distinct immune and tumour cell populations within the tissue where multiple markers are required. Bioinformatics pipeline of immunofluorescence results consists in a series of aggregations of "cell object" data retrieved from Halo AI analysis. Cell object data are provided in an Excel table file in which each row represents a cell and columns represent fluorescence results in each cell. For each spot, a percent of VISTA⁺ cells (VISTA⁺ cells correspond to cells whose fluorescence intensity is above a threshold) is calculated for each cell type (macrophage M2, TL CD8, tumour ...), then this percentage is aggregated by patients (several spots for a same patient).

VISTA, CD8, Cytokeratin, CD33, CD163 and PD-L1 positive cells were thus quantified and analysed across all the indication available in the Pierre Fabre's biobank.

### Results

Consistent with the literature, we have observed that VISTA is not only predominantly expressed in the TME but also frequently and highly expressed in tumour cells in some indications such as sarcoma, mesothelioma, kidney, and gastric cancer for example. In the TME, VISTA is found to be highly expressed in CD33 and CD163 populations, particularly in kidney cancer and sarcoma. Moreover, we found that tumour infiltrating CD8 T-cells often expressed VISTA, notably in sarcoma, kidney cancer, and hepatocellular carcinoma.

**Table 3: Scoring table: Top 6 indications ranking based on 7 different readouts**

| Readout | Ranking | | | | | |
|---|---|---|---|---|---|---|
| | 1 | 2 | 3 | 4 | 5 | 6 |
| % of VISTA+ cells | Sarcoma | Kidney | Gastric | Esophagus | Endometrial | Gallblabbder |
| Density of VISTA+ cells | Sarcoma | Gastric | Endometrial | Kidney | Lung (m) | Cervix |
| Intensity of VISTA+ cells | Sarcoma | Kidney | Gastric | Endometrial | TNBC | Esophagus |
| % of VISTA among CD8 pop | Sarcoma | Kidney | HCC | Endometrial | Gastric | Melanoma |
| % of VISTA among CD33 pop | Kidney | Melanoma | Esophagus | Endometrial | Sarcoma | H&N |
| % of VISTA among CD163 pop | Kidney | Gastric | Ovary | Sarcoma | Prostate | Lung ADC |
| % of VISTA among TC pop | Sarcoma | Kidney | Gastric | TNBC | Endometrial | Esophagus |
| | | | | | | |
| | | | | | | |
| | | | | | | |
| 1 | Kidney | 7 | | | | |
| 2 | Sarcoma | 7 | | | | |
| 3 | Endometrial | 6 | | | | |
| 4 | Gastric | 5 | | | | |
| 5 | Esophagus | 4 | | | | |
| 6 | Melanoma | 2 | | | | |

Based on %, density and intensity of VISTA and its coexpression with CD8, CD163, CD33 and tumour cells, Sarcoma, Kidney, Endometrial, Gastric, Oesophageal cancer and Melanoma appears to be relevant indications for anti-vista immunotherapy.

## Claims

1. A multiplex method of scoring a tumour tissue sample stained with a first immune checkpoint protein, comprising:
a) contacting the tissue sample with a primary antibody directed to the first immune checkpoint protein; and
b) contacting the same tissue sample with plurality of antibodies, the plurality of antibodies comprising:
• at least one antibody directed to a tumour cell-specific marker;
• at least one antibody directed to a cytotoxic T cell marker;
• at least one antibody directed to a M2 macrophage marker;
• at least one antibody directed to a myeloid-derived cell marker;
• at least one antibody directed to a second immune checkpoint protein;
c) visualising each of the antibodies in the tissue sample with a reagent that generates a detectable signal corresponding to each of the antibodies, wherein each of the detectable signals is distinguishable from the others;
d) quantifying the number of cells expressing VISTA for each of tumour cells, cytotoxic T cells, M2 macrophage, MDSCs, and cells expressing the second immune checkpoint protein; and
e) scoring the tumour based on this quantification.

2. The multiplex method of claim 1, wherein the first immune checkpoint protein is VISTA.

3. The multiplex method of any one of claims 1 or 2, wherein the tumour sample is a formalin-fixed paraffin embedded (FFPE) sample.

4. The multiplex method of any one of claims 1 to 3, wherein the tumour is a solid tumour.

5. The multiplex method of any one of claims 1 to 4, wherein:
- the tumour cell marker is cytokeratin,
- the cytotoxic T cell marker is CD8,
- the M2 macrophage marker is CD123,
- the MDSC marker is CD33, and/or
- the second immune checkpoint protein is PD-L1.

6. The multiplex method of any one of claims 1 to 5, wherein the antibody is labelled with a detectable label.

7. The multiplex method of claim 6, wherein the detectable label is a fluorescent label.

8. The multiplex method of claim 7, wherein step c) involves measuring information corresponding to one or more stains of each of the labelled antibodies directed against the first immune checkpoint protein, tumour cell-specific marker, the cytotoxic T cell marker, the M2 macrophage marker, the myeloid-derived cell marker, and the second immune checkpoint protein in the biological sample.

9. The multiplex method of claim 8, wherein step c) involves:
- generating a first image of the biological sample, wherein the first image corresponds to a pattern of staining of the first immune checkpoint protein in the biological sample;
- generating a second image of the biological sample, wherein the second image corresponds to a pattern of the tumour cell-specific marker in the biological sample;
- generating a third image of the biological sample, wherein the third image corresponds to a pattern of the cytotoxic T cell marker staining in the biological sample;
- generating a fourth image of the biological sample, wherein the fourth image corresponds to a pattern of the M2 macrophage marker staining in the biological sample;
- generating a fifth image of the biological sample, wherein the fifth image corresponds to a pattern of myeloid-derived cell marker staining in the biological sample; and
- generating a sixth image of the biological sample, wherein the sixth image corresponds to a pattern of staining of the second immune checkpoint protein in the biological sample.

10. A method for ranking a plurality of tissue samples, comprising the steps of:
a) scoring each of the tissue samples using the scoring method of any one of claims 1 to 9, and
b) ranking the tissue samples based on the scoring of a);
wherein the tissue samples are from the same tissue from different patients, from different tissues from different patients, or from different tissues from the same patient.

11. A method of prognosing an anticancer therapy in patient comprising:
a) taking a first tumour tissue sample from the patient at a first time point;
b) taking a second tumour tissue sample from the patient at a second time point;
c) scoring the first tumour sample from patient using the scoring of any one of claims 1 to 9, thereby generating a first score;
d) scoring the second tumour sample from the patient using the scoring of any one of claims 1 to 9, thereby generating a second score;
e) comparing the first score of c) and the second score of d), and
f) determining the prognosis of the patient based on the comparison of e).

12. The method of claim 10, the prognosis of the patient is bad when the second score of d) is higher than the first score of c).

13. The method of any one of claims 10 or 11, wherein the anticancer therapy is immunotherapy or chemotherapy.

14. A method for predicting a treatment response of a patient to a proposed treatment for a cancer comprising:
a) scoring a tumour tissue sample from the patient using the scoring of any one of claims 1 to 9, and
b) determining that the patient will or will not be responsive to the proposed treatment based on the score of the tumour tissue sample.

15. The method of claim 13, wherein the patient is predicted to be responsive to the treatment when at least one of tumour cells, cytotoxic T cells, M2 macrophages, and MDSCs expresses the first immune checkpoint protein in the tumour tissue sample.

16. The method of any ones of claims 13 or 14, wherein the treatment comprises administering an anti-VISTA monoclonal antibody 26A comprising 6 CDRs represented by SEQ ID NOS:3-8.

17. The method of claim 15, wherein the anti-VISTA monoclonal antibody 26A comprises the V_{H} and V_{L} represented by SEQ ID NO:9 and SEQ ID NO:10.
